# EUROPEAN PATENT SPECIFICATION

(11) **EP 1 458 377 B1**
(45) Date of publication and mention of the grant of the patent: **22.03.2006**
(21) Application number: 02796711.6
(22) Date of filing: 20.12.2002
(51) Int. Cl.: A61K 31/404, A61K 9/20

(54) **TEGASEROD PHARMACEUTICAL COMPOSITIONS**
PHARMAZEUTISCHE ZUSAMMENSETZUNGEN ENTHALTEND TEGASEROD
COMPOSITIONS PHARMACEUTIQUES RENFERMANT TEGASEROD

(30) Priority: 21.12.2001 EP 01403339
(43) Date of publication of application: 22.09.2004
(73) Proprietor: Novartis AG, 4056 Basel (CH); Novartis Pharma GmbH, 1230 Wien (AT)
(72) Inventor: AUBERT, Jérome, F-74800 La Roche sur Foron (FR); VITZLING, Christian, F-75012 Paris (FR)
(74) Representative: de Weerd, Petrus G.W.
(86) International application number: PCT/EP2002/014674
(87) International publication number: WO 2003/053432

(56) References cited:
- WO-A-00/10526
- WO-A-00/57857

## Description

### Field of the invention

The present invention relates to pharmaceutical compositions for administering tegaserod and to processes for manufacturing such compositions.

### Background of the Invention

Tegaserod (3-(5-methoxy-1H-indol-3-yl-methylene)-N-pentylcarbazimidamide) and pharmaceutically acceptable salts thereof are known from EP 505322 and under the trade marks ZELMAC and ZELNORM. Published PCT Application WO 00/10526 describes tegaserod compositions, e.g. solid oral pharmaceutical compositions and use in anal incontinence.

Despite the merits of the above-mentioned compositions, there remains a need for more economic and stable compositions which can be formulated effectively.

### Summary of the invention

In one aspect this invention provides a solid pharmaceutical composition for oral adminstration comprising
tegaserod in base or salt form in an amount of up to 10% by weight,
a diluent in an amount of 70 to 90% by weight, and
a disintegrant in an amount of less than 15% by weight,
wherein the amounts by weight are based on the total weight of the composition.

The term "disintegrant" is understood to mean a substance or mixture of substances which facilitates disintegration of the composition after administration in order that the active ingredient be released from the composition as efficiently as possible to allow for its rapid dissolution (see e.g. "Remington's Pharmaceutical Science" 18th edition (1990), "The Theory and Practice of Industrial Pharmacy" Lachman et al. Lea & Febiger (1970)).

The active agent used in compositions according to the present invention is tegaserod, a serotonergic active agent acting on the gastro-intestinal system as partial agonist of the 5-HT₄ receptor. It is poorly soluble and acid sensitive. The active agent is preferably in salt form, e.g., hydrogen maleate or hydrochloride, or in free form.

5-HT₄ receptor partial agonists are useful for the prevention and treatment of gastro-intestinal motility disorders, e.g., Irritable Bowel Syndrome (IBS), Gastro-Esophageal Reflux Disease (GERD), Functional Dyspepsia (FD), Post Operative lleus (POI), Diabetic gastroporesis and chronic constipation.

Tegaserod is a 5-HT₄ partial agonist of formula 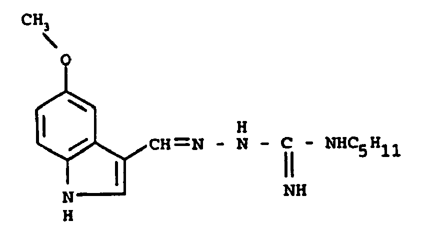 or pharmaceutically acceptable salt form thereof, e.g. the hydrogen maleate (hereinafter "hml") salt. Tegaserod has a solubility of about 0.02% at 25°C in water and is acid sensitive. We have found that compositions thereof may be produced which provide good dissolution even in the stomach.

In one embodiment, the composition of the invention comprises less than 15%, e.g. less than 14%, preferably 12% or less, e.g. about 10% or less, e.g. 5 to 10% by weight of disintegrant based on the total weight of the composition. We have observed that the use of such a low disintegrant content improves the dissolution rate.

The diluent may comprise lactose, mannitol, sucrose, calcium sulphate, calcium phosphate or microcrystalline cellulose (MCC USP (AvicelTM PH-102, FMC Corp.) The diluent may be present in an amount from 50 to 90 %, preferably from 70 to 90 % more preferably from 75 to 85%. Preferably the diluent is lactose, more preferably as α-lactose monohydrate and/or as amorphous material (Spray dried lactoseTM, Formost Corp.).

As disintegrant the composition of the present invention may comprise:
- crospovidone (e.g. with molecular weight >10⁶ Daltons), e.g. Polyplasdone XL® Kollidon CL ®, Polyplasdone XL-10®,
- pregelatinized starch (e.g. with MW 30000-120000 Daltons), e.g., starch 1500™ (Colorcon UK).

Preferably, the disintegrant is crospovidone which is preferably water insoluble. Ideally the disintegrant rapidly exhibits high capillary or pronounced hydration capacity with little tendency to gel formation.
The particle size of the disintegrant may be from about 1 to about 500 micrometers. A preferred particle size distribution is from 10 to 400 e.g. less than 400 micrometers, e.g., for Polyplasdone XL® , less than 80 micrometers, e.g., less than 74 micrometers for, e.g., Polyplasdone XL-10®, approximately 50% greater than 50 micrometers and maximum of 1% greater than 250 micrometers in size for, e.g., Kollidon CL® . A preferred crospovidone is Polyplasdone XL®, e.g., with a density of about 0.213 g/cm³ (bulk) or 0.273 g/cm³ (tapped).

The preferred crospovidone content of the composition is from about 8% to about 14%, most preferably from about 9% to about 12%, by weight.

The composition of the present invention may further comprise a glidant e.g. Colloidal silicon dioxide (Aerosil, Degussa). From about 0.05% to about 1% by weight of glidant may be used, e.g. about 0.1 % of Aerosil or similar.

The composition may further comprise one or more lubricants, e.g., in an amount within the range of from 3 to 8%, e.g. from 5 to 7% by weight of the composition.

Examples of such lubricants include:
- magnesium stearate (Faci),
- sodium benzoate
- glyceryl mono fatty acid, e.g. having a molecular weight of from 200 to 800 Daltons e.g. gylceryl monostearate (e.g., Danisco, UK),
- glyceryl behenate (e.g., CompritolATO888™, Gattefossé France)
- glyceryl palmito-stearic ester (e.g. Precirol™, Gattefossé France)
- polyoxyethylene glycol (PEG, BASF)
- hydrogenated cotton seed oil (Lubitrab, Edward Mendell Co Inc),
- castor seed oil (Cutina HR, Henkel)

In a preferred embodiment the lubricant is glyceryl behenate. We have observed that the use of glyceryl behenate improves lubrication properties, avoids tablet adhesion and helps stabilise the composition. Further there is no or negligible impact on tegaserod in vitro dissolution rate and tablet disintegration of the composition. Preferably the amount of glyceryl behenate used is about 6% by weight.

Glyceryl behenate typically comprises mixtures of glyceryl behenate and glyceryl dibehenate. For the purposes of the present description the term "glyceryl behenate" is used to indicate mixtures of glyceryl behenate and glyceryl dibehenate and also each component separately, i.e. glyceryl behenate or glyceryl dibehenate; for instance in line with the nomenclature used in monograph USP24/NF19.

The composition of the invention may comprise one or more binders, e.g., in an amount in the range of from 1 to 10%, e.g., 2 to 8%, e.g. about 5% by weight. Particularly the following binders may be used:
- hydroxy-propyl-methyl cellulose (HPMC2910, Pharmacoat603TM, Shin-Etsu Chemical Co Ltd)
- copolyvidone (KollidonTM VA64, BASF)
- potato starch, wheat starch, com starch, e.g., having a molecular weight of from 30000 to 120000,
or a mixture thereof. Preferably from about 1% to about 10%, e.g. about 4% to about 6%, by weight of hydroxy propylmethyl cellulose is used as binder. In accordance with the invention we have observed that the presence of hydroxy propylmethyl cellulose improves dissolution of tegaserod even in the presence of a low amount of disintegrant.

Other conventional excipients which may optionally be present in the composition of the invention include preservatives, stabilisers, anti-adherents or silica flow conditioners or glidants, e.g., silicon dioxide (e.g., Syloid® , Aerosil® ) as well as FD&C colours such as ferric oxides.

Other excipients disclosed in the literature, as for instance in Fiedler's "Lexicon der Hilfstoffe", 4th Edition, ECV Aulendorf 1996 and "Handbook of Pharmaceutical Excipients" Wade and Weller Ed.(1994) may be used in the pharmaceutical compositions according to the invention.

A preferred composition of the invention may comprise from about 0.5 to 15% by weight of tegaserod; less than 15 % by weight of disintegrant e.g. crospovidone; from 3 to 7% by weight of lubricant, e.g. glyceryl behenate; from 50 to 90% by weight of diluent, e.g. lactose; from 0.1 % to 1% by weight of glidant, and optionally from 1 to 10% of binder, e.g. hydroxypropylmethyl cellulose (HPMC).

The compositions of this invention may be free or substantially free of surfactant.

In a further aspect the present invention provides an oral, e.g. tablet composition comprising the active agent tegaserod.

Daily dosages required in practising the method of the present invention will vary depending upon, for example the mode of administration and the severity of the condition to be treated. An indicated daily dose is in the range of from about 1 to about 30 mg, e.g. from 2 to 24 mg,of active agent for oral use, conveniently administered once or in divided dosages.

In one embodiment the present invention provides a round shaped tablet with a diameter of 6 to 10 mm, preferably 7 mm.

In a further aspect the present invention provides a process for the production of the compositions of the invention. The compositions of the invention may be prepared by working up active agent with excipients. The composition of the invention may be formed into tablets by processes involving granulation, especially under dry conditions. Advantageously the composition of the invention may be formed into tablets by direct compression. The following processes A, B and C are contemplated:

### Process A

The composition of the invention may be obtained by
(i) preparing a mixture of tegaserod, diluent and lubricant,
(ii) sieving the mixture
(iii) adding the disintegrant, glidant, lubricant and optionally binder and blending the sieved mixture of step (ii) and
(iv) forming tablets by direct compression.

Part of the lubricant may be added in the mixture of step (i), the rest in the final mixture of step (iii) or the total amount of lubricant may be added in the final mixture of step (iii).

The resulting powder blends of step iii) are compressed on either a single punch press (Korsh EKO), 6 station-rotary press (Korsh PH106), 17 station-rotary press (Korsh PH 230) or 43 station-rotary press (Fette PT2090).

All components may be mixed together, sieved through and mixed again. Tablets are then formed by direct compression.

### Process B

The compositions of the invention may be obtained by
(i) preparing a mixture of Tegaserod and diluent,
(ii) sieving the mixture,
(iii) adding the disintegrant, glidant and optionally binder and blending the sieved mixture of step (ii), and
(iv) adding the lubricant by spray lubrication when forming tablets by direct compression.

A 43 station rotary press (Fette PT 2090) with a magnesium stearate spraying system may be conveniently used to carry out step (iv).

The components may be mixed together, sieved and mixed again. The lubricant is added by spray lubrication when the tablets are formed by direct compression.

### Process C

In another embodiment the compositions of the invention may be obtained by
(i) preparing a mixture of Tegaserod, diluent, disintegrant, glidant and optionally binder,
(ii) compacting the mixture of step (i) by roller compaction,
(iii) milling the mixture of step (ii), and
(iv) forming tablets by compression or adding the lubricant by spray lubrication when forming tablets by compression.

Tablets may be formed by compressing the resulting powder on a single punch press (Korsh EKO), 6 station-rotary press (Korsh PH106), 17 station-rotary press (Korsh PH 230), a 43 station-rotary press (Fette PT2090) or a 43 station rotary press (Fette PT 2090) with the magnesium stearate spraying system.

The following is a description by way of example only of compositions and processes of the invention.

### Example 1

A 6 mg tablet is prepared using the direct compression method.

| Component | Quantitiy (125 mg tablet) %w/w |
|---|---|
| Tegaserod maleate | 6.65 |
| Lactose spray dried | 82.85 |
| Crospovidone | 6.00 |
| Aérosil | 0.50 |
| glyceryl behenate | 4.00 |

A blend is formed by mixing tegaserod maleate, lactose, crospovidone, aérosil and glyceryl behenate. This blend is sieved and the mixture is blended again. The resulting powder blends are compressed using a 17 station-rotary press (Korsh PH 230) equipped with 7 mm, round upper punches.

### Example 2

A 6 mg tablet is prepared using the direct compression method.

| Component | Quantitiy (125 mg tablet) % w/w |
|---|---|
| Tegaserod maleate | 6.65 |
| Lactose spray dried | 72.25 |
| hydroxy propylmethyl cellulose | 5 |
| Crospovidone | 10.00 |
| Aérosil | 0.10 |
| glyceryl behenate | 6.00 |

A preblend is formed by mixing tegaserod maleate, hydroxy propylmethyl cellulose, a part of glyceryl behenate and a part of lactose. This preblend is mixed with the remaining excipients except glyceryl behenate.This blend is lubricated with the remaining part of glyceryl behenate.
The final blend is compressed using a rotary press (Korsh PH 343 or Fette PT2090) equipped with 7 mm, round upper punches.

### Example 3

A 6 mg tablet is prepared using the direct compression method with *in situ spray* lubrication.

| Component | Quantitiy (125 mg tablet) % w/w |
|---|---|
| Tegaserod maleate | 6.65 |
| Lactose spray dried | 84.85 |
| HPMC | 3.00 |
| Crospovidone | 5.00 |
| Aérosil | 0.50 |
| Magnesium stearate | < 0.3 |

A blend is formed by mixing tegaserod maleate, lactose, crospovidone, aérosil and compritol. This blend and the mixture is blended again. The lubricant magnesium stearate is added by spray lubrication. The resulting powder blends are compressed using a 43 station-rotary press (Fette PT 2090) equipped with 7 mm, round upper punches.

### Example 4

A 6 mg tablet is prepared using roller compaction

| Component | Quantitiy (125 mg tablet) % w/w |
|---|---|
| Tegaserod maleate | 6.65 |
| Lactose spray dried | 76.85 |
| Crospovidone | 10.00 |
| Aérosil | 0.50 |
| glyceryl behenate | 6.00 |

Compositions are prepared by mixing tegaserod maleate, lactose, crospovidone, aérosil and glyceryl behenate. This mixture is compacted by roller compaction and milled. Tablets are formed by compression.

The present invention thus provides a solid oral pharmaceutical composition comprising a 5-HT₄-receptor partial agonist and a lower amount of disintegrant than hitherto used. Comparative Example

The compositions of the present invention typically have the following advantages compared to the compositions described in WO 00/10526:
A - Tablets manufactured according to the present invention are less hygroscopic than tablets manufactured according to WO 00/10526 (wet granulation process):
Tablets (6mg tegaserod) are manufactured as described in Example 2 above and also as described in Example 3 of WO 00/10526. Tablets from both batches are exposed to a 60% relative humidity atmosphere at 25°C for a period of 72 hours. The weight increase of the tablets is measured and the percentage weight increase due to absorption of water vapour is calculated. The results obtained are given below.

| Tablet type | Water uptake in % by wt after exposure of non protected 6 mg tablets at 25°C/60 % r.h. for 72 hours |
|---|---|
| Example 2 of the present invention : direct compression | approx. 2 % |
| Example 3 of W O 00/10526 (wet granulation) | approx 5 % |

B - Tablets manufactured according to the present invention (eg example 2: direct compression) have similar tegaserod dissolution profiles to tablets manufactured according to WO 00/10526 (wet granulation process):
Tablets (6mg tegaserod) prepared according to Example 2 of the present application and according to Example 3 of WO 00/10526 are suspended in 900ml aliquots of aqueous buffers at various pHs, and in tap water, with agitation by rotating paddle (50 rpm). The percentage dissolution of tegaserod, after suspension treatment for 30 minutes, is calculated for each tablet type, for each treatment regime. The results obtained are given below.
6 mg tablet ; percentage of tegaserod dissolved after 30 minutes (rotating paddle, 50 rpm)

| Tablet type | USP buffer pH 4.5 (900 ml) | USP buffer pH 6.5 (900 ml) | USP buffer pH 7.5 (900 ml) | water (500 ml) |
|---|---|---|---|---|
| Example 2 of the present invention : direct compression | 19.4 | 94.8 | 91.6 | 96.4 |
| Example 3 of WO 00/10526 | 19.9 | 98.1 | 95.7 | 99.0 |

C - the manufacturing processes described in the present invention are simpler, shorter and cheaper than the process described in WO 00/10526 (wet granulation)

The invention provides tegaserod compositions with fewer components than hitherto known and a simple dry process without granulation. The formulations of the present invention are less hygroscopic, overcome adhesion problems and provide complete or substantially complete dissolution within 30 minutes.

## Claims

1. A solid pharmaceutical composition for oral adminstration comprising
tegaserod in base or salt form in an amount of up to 10% by weight,
a diluent in an amount of 70 to 90% by weight,
a disintegrant in an amount of less than 15% by weight, and
wherein the amounts by weight are based on the total weight of the composition.

2. A composition as claimed in claim 1 wherein tegaserod is in the form of the maleate salt.

3. A composition as claimed in claim 1 or 2 further comprising a glidant.

4. A composition as claimed in any preceding claim further comprising a lubricant.

5. A composition as claimed in any preceding claim further comprising a binder.

6. A composition as claimed in any preceding claim wherein the diluent is selected from the group consisting of lactose, mannitol, sucrose, calcium phosphate or microcrystalline cellulose.

7. A composition as claimed in any preceding claim wherein the diluent is lactose.

8. A composition as claimed in any preceding claim wherein the disintegrant is present in an amount of 12% or less by weight based on the total weight of the composition.

9. A composition as claimed in any preceding claim wherein the disintegrant is crospovidone.

10. A pharmaceutical composition as claimed in any of claims 4 to 9 claim wherein the lubricant is present in an amount of 3 to 7% based on the total weight of the composition.

11. A pharmaceutical composition as claimed in any of claims 4 to 10 wherein the lubricant is glycerol monostearate or glycerol behenate.

12. A pharmaceutical composition as claimed in any of claims 3 to 11 wherein the glidant is colloidal silica dioxide.

13. A pharmaceutical composition in any of claims 3 to 12 wherein the the binder is hydroxy propylmethyl cellulose.

14. A process for the production of a composition as claimed in any preceding claim which process is carried out under substantially dry conditions using granulation.

15. A process for the production of a composition as claimed in any preceding claim which process comprises:
(i) preparing a mixture of tegaserod, diluent and lubricant,
(ii) sieving the mixture,
(iii) adding the disintegrant, glidant and optionally binder and blending the sieved mixture of step (ii), and
(iv) forming tablets by direct compression.

16. A process for the production of a composition as claimed in claim 15 wherein the components are mixed with tegaserod, sieved and mixed again before tabletting.

17. A process for the production of a composition as claimed in any of claims 1 to 13 which process comprises:
(i) preparing a mixture of tegaserod, and diluent
(ii) sieving the mixture
(iii) adding the disintegrant, glidant and optionally binder and blending the sieved mixture of step (ii)
(iv) adding the lubricant by spray lubrication when forming tablets by direct compression.

18. A process for the production of a composition as claimed in claim 17 wherein all the components are mixed with tegaserod, sieved through and mixed again before tabletting.

19. A process for the production of a composition as claimed in any of claims 1 to 13 which process comprises:
(i) preparing a mixture of tegaserod, diluent, disintegrant, glidant and optionally binder
(ii) compacting the premix of step (i) by roller compaction
(iii) milling the mixture of step (ii) and
(iv) forming tablets by compression.

## Patentansprüche

1. Feste pharmazeutische Zusammensetzung zur oralen Verabreichung, umfassend
Tegaserod als Base oder in Form eines Salzes in einer Menge von bis zu 10 Gew.-%,
ein Verdünnungsmittel in einer Menge von 70 bis 90 Gew.-% und
ein Zerfallhilfsmittel in einer Menge von weniger als 15 Gew.-%,
worin die Gewichtsmengen auf das Gesamtgewicht der Zusammensetzung bezogen sind.

2. Zusammensetzung nach Anspruch 1, worin Tegaserod in Form des Maleatsalzes vorhanden ist.

3. Zusammensetzung nach Anspruch 1 oder 2, weiter umfassend ein Gleitmittel.

4. Zusammensetzung nach einem der vorhergehenden Ansprüche, weiter umfassend ein Schmiermittel.

5. Zusammensetzung nach einem der vorhergehenden Ansprüche, weiter umfassend ein Bindemittel.

6. Zusammensetzung nach einem der vorhergehenden Ansprüche, worin das Verdünnungsmittel aus der Gruppe ausgewählt ist, die besteht aus Lactose, Mannit, Saccharose, Calciumphosphat oder mikrokristalliner Cellulose.

7. Zusammensetzung nach einem der vorhergehenden Ansprüche, worin das Verdünnungsmittel Lactose ist.

8. Zusammensetzung nach einem der vorhergehenden Ansprüche, worin das Zerfallhilfsmittel in einer Menge von 12 % oder weniger, bezogen auf das Gesamtgewicht der Zusammensetzung, vorhanden ist.

9. Zusammensetzung nach einem der vorhergehenden Ansprüche, worin das Zerfallhilfsmittel Crospovidon ist.

10. Pharmazeutische Zusammensetzung nach einem der Ansprüche 4 bis 9, worin das Schmiermittel in einer Menge von 3 bis 7 %, bezogen auf das Gesamtgewicht der Zusammensetzung, vorhanden ist.

11. Pharmazeutische Zusammensetzung nach einem der Ansprüche 4 bis 10, worin das Schmiermittel Glycerinmonostearat oder Glycerinbehenat ist.

12. Pharmazeutische Zusammensetzung nach einem der Ansprüche 3 bis 11, worin das Gleitmittel kolloidales Siliciumdioxid ist.

13. Pharmazeutische Zusammensetzung nach Anspruch 3 bis 12, worin das Bindemittel Hydroxypropylmethylcellulose ist.

14. Verfahren zur Herstellung einer Zusammensetzung nach einem der vorhergehenden Ansprüche, welches unter praktisch trockenen Bedingungen und Anwendung einer Granulation durchgeführt wird.

15. Verfahren zur Herstellung einer Zusammensetzung nach einem der vorhergehenden Ansprüche, wobei dieses Verfahren umfasst:
(i) Herstellung eines Gemisches aus Tegaserod, Verdünnungsmittel und Schmiermittel,
(ii) Sieben des Gemisches,
(iii) Zusatz des Zerfallhilfsmittels, Gleitmittels und optional Bindemittels und Vermischung des gesiebten Gemisches der Stufe (ii) und
(iv) Bildung von Tabletten durch direkte Kompression.

16. Verfahren zur Herstellung einer Zusammensetzung nach Anspruch 15, worin die Komponenten mit Tegaserod vermischt werden, das Gemisch gesiebt wird und vor einer Tablettierung erneut vermischt wird.

17. Verfahren zur Herstellung einer Zusammensetzung nach einem der Ansprüche 1 bis 13, wobei dieses Verfahren umfasst:
(i) Herstellung eines Gemisches aus Tegaserod und Verdünnungsmittel,
(ii) Sieben des Gemisches,
(iii) Zusatz des Zerfallhilfsmittels, Gleitmittels und optional Bindemittels und Vermischung des gesiebten Gemisches der Stufe (ii) und
(iv) Zusatz des Schmiermittels durch Sprühschmierung während der Bildung von Tabletten durch eine direkte Kompression.

18. Verfahren zur Herstellung einer Zusammensetzung nach Anspruch 14, worin alle Komponenten mit Tegaserod vermischt, gesiebt und vor einer Tablettierung erneut gesiebt werden.

19. Verfahren zur Herstellung einer Zusammensetzung nach einem der Ansprüche 1 bis 13, wobei dieses Verfahren umfasst:
(i) Herstellung eines Gemisches aus Tegaserod, Verdünnungsmittel, Zerfallhilfsmittel, Gleitmittel und optional Bindemittel,
(ii) Kompaktierung des Vorgemisches der Stufe (i) durch Walzenkompaktion,
(iii) Vermahlung des Gemisches der Stufe (ii) und
(iv) Bildung von Tabletten durch Kompression.

## Revendications

1. Composition pharmaceutique solide destinée à une administration par voie orale comprenant :
- du tegaserod sous forme d'une base ou d'un sel selon une quantité allant jusqu'à 10% en poids,
- un diluant selon une quantité allant de 70 à 90% en poids,
- un délitant selon une quantité inférieure à 15% en poids
et **caractérisée en ce que** ces quantités sont exprimées en poids sur base du poids total de la composition.

2. Composition selon la revendication 1, **caractérisée en ce que** le tegaserod est présent sous forme d'un sel de maléate.

3. Composition selon la revendication 1 ou 2 comprenant également un glissant.

4. Composition selon l'une quelconque des revendications précédentes comprenant également un lubrifiant.

5. Composition selon l'une quelconque des revendications précédentes comprenant également un liant.

6. Composition selon l'une quelconque des revendications précédentes, **caractérisée en ce que** le diluant est sélectionné à partir du groupe composé de lactose, de mannitol, de saccharose, de phosphate de calcium ou de cellulose microcristalline.

7. Composition selon l'une quelconque des revendications précédentes, **caractérisée en ce que** le diluant est du lactose.

8. Composition selon l'une quelconque des revendications précédentes, **caractérisée en ce que** le délitant est présent selon une quantité de 12% ou moins sur base du poids total de la composition.

9. Composition selon l'une quelconque des revendications précédentes, **caractérisée en ce que** le délitant est du crospovidone.

10. Composition selon l'une quelconque des revendications 4 à 9, **caractérisée en ce que** le lubrifiant est présent selon une quantité allant de 3 à 7% en poids sur base du poids total de la composition.

11. Composition selon l'une quelconque des revendications 4 à 10, **caractérisée en ce que** le lubrifiant est du monostéarate de glycérol ou du béhénate de glycérol.

12. Composition selon l'une quelconque des revendications 3 à 11, **caractérisée en ce que** le glissant est du dioxyde de silicium colloïdal.

13. Composition selon l'une quelconque des revendications 3 à 12, **caractérisée en ce que** le liant est de l'hydroxy propylméthyle cellulose.

14. Procédé pour la production d'une composition tel que revendiqué à l'une quelconque des revendications précédentes, **caractérisé en ce que** le procédé est réalisé sous des conditions sèches à l'aide d'un procédé de granulation.

15. Procédé pour la production d'une composition tel que revendiqué à l'une quelconque des revendications précédentes consistant :
(i) à préparer un mélange comprenant du tegaserod, un diluant et un lubrifiant,
(ii) à tamiser le mélange,
(iii) à ajouter le délitant, le glissant et, éventuellement, le liant et à mélanger le mélange tamisé de l'étape (ii), et
(iv) à former des comprimés par compression directe.

16. Procédé pour la production d'une composition tel que revendiqué à la revendication 15, **caractérisé en ce que** tous les composants sont mélangés avec du tegaserod, tamisés et à nouveau mélangés avant transformation en comprimés.

17. Procédé pour la production d'une composition selon l'une des revendications 1 à 13 consistant :
(i) à préparer un mélange comprenant du tegaserod et un diluant,
(ii) à tamiser le mélange,
(iii) à ajouter le délitant, le glissant et, éventuellement, le liant et à mélanger le mélange tamisé de l'étape (ii), et
(iv) à ajouter le lubrifiant par graissage par pulvérisation tout en formant des comprimés par compression directe.

18. Procédé pour la production d'une composition tel que revendiqué à la revendication 15, **caractérisé en ce que** tous les composants sont mélangés avec du tegaserod, tamisés et à nouveau mélangés avant transformation en comprimés.

19. Procédé pour la production d'une composition selon l'une des revendications 1 à 13 consistant :
(i) à préparer un mélange comprenant du tegaserod, un diluant, un délitant, un glissant et, éventuellement, un liant
(ii) à compacter le pré-mélange de l'étape (i) à l'aide d'un rouleau compresseur.,
(iii) à broyer le mélange de l'étape (ii), et
(iv) à former des comprimés par compression.
